# EUROPÄISCHE PATENTSCHRIFT

(11) **EP 4 137 554 B1**
(45) Veröffentlichungstag und Bekanntmachung des Hinweises auf die Patenterteilung: **02.10.2024**
(21) Anmeldenummer: 21191867.7
(22) Anmeldetag: 18.08.2021
(51) Int. Cl.: B01D 33/37, C12M 1/00, B01D 33/21, B01D 63/16, B01D 65/08

(54) **FERMENTEREINRICHTUNG**
FERMENTATION DEVICE
AGENCEMENT DE FERMENTEUR

(43) Veröffentlichungstag der Anmeldung: 22.02.2023
(73) Patentinhaber: ANDRITZ Separation GmbH, 85256 Vierkirchen (DE)
(72) Erfinder: GRIM, Gunnar, 85258 Weichs (DE)
(74) Vertreter: Viering, Jentschura & Partner mbB Patent- und Rechtsanwälte

(56) Entgegenhaltungen:
- EP-A1- 1 236 500
- EP-A1- 2 905 067
- WO-A1-2011/050825
- WO-A2-02/05935
- DE-A1- 10 004 096
- DE-A1- 10 154 549
- DE-A1- 19 502 848
- DE-A1- 2 251 171
- DE-U1- 202005 018 806

## Beschreibung

Die Erfindung betrifft eine Fermentereinrichtung mit einer Querstromfiltrationseinrichtung, ein System, welches eine solche Fermentereinrichtung aufweist, und ein Verfahren zum Betreiben einer solchen Fermentereinrichtung.

Bei dynamischer Querstromfiltration werden Filtrationsmembranen relativ zu einer in einem Behälter aufgenommenen Suspension bewegt, um durch einen entstehenden Suspensions-Querstrom entlang der Filtermembranen während des laufenden Filtrationsbetriebs die Filtermembranen von Feststoffrückständen frei zu halten (das heißt, es wird in der Fermentationsbrühe an der Oberfläche der jeweiligen Filtermembran eine Querströmung erzeugt). Eine dynamische Querstromfiltrationsvorrichtung ist beispielsweise aus der DE 10 2014 101 499 A1 bekannt. Aus der WO 2011 050 825 A1 ist es ferner z.B. bekannt, eine Querstromfiltrationsvorrichtung in Verbindung mit einer Fermentereinrichtung zu verwenden, um beispielsweise Zielprodukte und/oder Metabolismusprodukte aus einer Fermentationsbrühe zu gewinnen.

EP 2 905 067 bezieht sich auf eine dynamische Querstromfiltrationseinrichtung mit Filtrationsmembranen, die relativ zu einer im Behälter aufgenommenen Suspension bewegt werden, um durch einen entstehenden Suspensions-Querstrom entlang der Filtermembranen während des laufenden Filtrationsbetrieb die Filtermembranen von Filterrückständen frei zu halten, da an der Oberfläche der jeweiligen Filtermembran eine Querströmung erzeugt wird.

Durch die Erfindung wird eine Fermentereinrichtung geschaffen, mittels welcher ein verbesserter Fermentationsbetrieb und damit ggf. eine verbesserte Effizienz (z.B. in Form einer erhöhten Ausbeute) in der Gewinnung von Zielprodukten aus einer Fermentationsbrühe erzielt werden kann. Durch die Erfindung werden ferner ein Fermenter-System mit einer solchen Fermentereinrichtung und ein Verfahren zum Betreiben einer solchen Fermentereinrichtung geschaffen.

Hierzu stellt die vorliegende Erfindung eine Fermentereinrichtung gemäß dem Anspruch 1, ein Fermenter-System gemäß dem Anspruch 12 und ein Verfahren zum Betreiben einer Fermentereinrichtung gemäß dem Anspruch 13 bereit. Weiterbildungen der Erfindung sind in den jeweiligen abhängigen Ansprüchen beschrieben.

Gemäß dem Gegenstand des Anspruchs 1 ist es möglich, ein Versorgungsgas, z.B. Sauerstoff, z.B. reiner Sauerstoff, und/oder sauerstoffhaltige Luft und/oder Stickstoff (z.B. reiner Stickstoff oder stickstoffhaltige Luft), über die mit der Versorgungsgasquelle verbundene wenigstens eine von der jeweiligen Körperverbindungsleitung via die jeweils hierzu zugeordnete Filtermembran in das Innere des Fermenterbehälters zuzuführen. Indem die Filtermembran(en) üblicherweise viele kleine Poren hat (haben), kann das Versorgungsgas damit sehr sanft und kleinblasig (z.B. in Form von Mikro- oder sogar Nanobläschen) in eine im Fermenterbehälter vorliegende Fermentationsbrühe eingebracht werden, so dass das Gas besser von der Fermentationsbrühe aufnehmbar ist (z.B. besser darin lösbar ist) und damit die Bildung größerer Versorgungsgasblasen, die sich dann z.B. an einer Behälteroberseite akkumulieren, bzw. eine Koaleszens von Versorgungsgasbläschen besser unterdrückt und/oder sogar im Wesentlichen verhindert werden kann. Damit können in der Fermentationsbrühe üblicherweise vorliegende Organismen/Zellen, wie z.B. Bakterien, Pilze, Algen, sonstige biologischen Zellkulturen, etc., besser mit einem für diese lebensnotwendigen Versorgungsgas versorgt werden, sodass die Fermentereinrichtung aufgabengemäß verbessert betreibbar ist. Die in der Fermentationsbrühe vorliegenden Organismen/Zellen/Enzyme erzeugen im Betrieb/Filtrationsbetrieb die gewünschten zur Weiterverwendung/Weiternutzung vorgesehen Zielprodukte, wie z.B. Metabolismusprodukte, wobei aufgrund der verbesserten Versorgung mit Versorgungsgas mit einer verbesserten Effizienz in der Erzeugung der Zielprodukte und ggf. auch mit einer verbesserten Lebensdauer der Arbeits-Organismen/Enzyme gerechnet werden kann.

Durch die Bewegung des jeweiligen Filtermembrankörpers kann ferner z.B. eine Verbesserung beim Verteilen der Versorgungsgasbläschen in der Fermentationsbrühe erzielt werden. Dies kann z.B. ferner dem Verhindern von Koaleszenz der Gasbläschen dienen, wodurch die Gasbläschen z.B. länger in der Fermentationsbrühe bleiben und z.B. eine verbesserte Gastransferrate erzielt werden kann.

Der Fermenterbehälter ist z.B. ein geschlossener Behälter, mit z.B. einer von einem Behälterdeckel verschlossenen Behälteröffnung für den Zugang zum Behälterinnenraum. Der jeweilige Filtermembrankörper kann irgendeine Form haben, wie z.B. eine Blockform oder eine Plattenform, wobei sich in der Praxis z.B. eine Scheibenform zur Erzeugung des gewünschten Querstroms entlang der Membranfläche als geeignet gezeigt hat. Der jeweilige Filtermembrankörper ist z.B. taumelnd bewegbar und/oder vibrationsbewegbar und/oder drehbar/rotierbar angeordnet und entsprechend taumelnd, vibrierend und/oder rotierend antreibbar mittels einer entsprechenden Antriebsvorrichtung, die z.B. Bestandteil der Fermentereinrichtung ist.

Es können z.B. mehrere Permeatabführleitungen vorgesehen sein, die der jeweiligen Körperverbindungsleitung jeweils einzeln zugeordnet sind, oder es können pro Permeatabführleitung zwei oder mehre Körperverbindungsleitungen daran angeschlossen sein. Es kann aber auch nur eine Permeatabführleitung vorgesehen sein, an welche dann die jeweilige Körperverbindungsleitung angeschlossen ist.

Im Falle der Ausbildung der jeweiligen Körperverbindungsleitung als Drehwelle, wie z.B. gemäß Anspruch 2, können z.B. immer zwei Körperverbindungsleitungen parallel nebeneinander mit gegenseitig kämmend ineinandergreifenden Filtermembrankörpern, z.B. Filtermembranscheiben, angeordnet sein. Es können auch z.B. vier oder immer vier Körperverbindungsleitungen parallel nebeneinander mit gegenseitig kämmend ineinandergreifenden Filtermembrankörpern, z.B. Filtermembranscheiben, angeordnet sein.

Gemäß der Ausführungsform von Anspruch 3, erste Option, kann über die jeweilige Körperverbindungsleitung, z.B. Scheibenverbindungsleitung, sowohl Permeat abgeführt, als auch Versorgungsgas zugeführt werden. Dabei kann z.B. zeitlich versetzt über die gleichen Filtermembranen Permeat abgeführt und Versorgungsgas zugeführt werden. Hierbei kann z.B. durch die Zufuhr von Versorgungsgas über die jeweilige an die (z.B. jeweilige) Permeatabführleitung angeschlossene Filtermembran eine zusätzliche Reinigung der Filtermembranen von daran anhaftenden Feststoffpartikeln erzielt werden. Es kann z.B. jede Körperverbindungsleitung sowohl mit der (z.B. jeweiligen) Permeatabführleitung als auch mit der Versorgungsgasquelle fluidverbunden sein (z.B. über ein Ventil oder mehrere Ventile) oder fluidverbindbar eingerichtet sein (z.B. über ein Ventil oder mehrere Ventile). Es können aber auch nur eine Anzahl von Körperverbindungsleitungen mit der Versorgungsgasquelle fluidverbunden und/oder fluidverbindbar sein, und nur eine Anzahl von Körperverbindungsleitungen mit der Permeatabführleitung verbunden und/oder fluidverbindbar sein. Im Falle der Ausführung von Anspruch 3, Option 2, kann z.B. zugleich (z.B. gleichzeitig) Versorgungsgas in den Fermenterbehälter zugeführt (über die jeweilige mit der Versorgungsgasquelle verbundene aber nicht mit der (z.B. jeweiligen) Permeatabführleitung verbundene oder verbindbare Körperverbindungsleitung) und Permeat aus dem Fermenterbehälter abgeführt werden (über die jeweilige mit der (z.B. jeweiligen) Permeatabführleitung verbundene Körperverbindungsleitung).

Die Ausführungsform gemäß dem Anspruch 4 kann z.B. dabei helfen, eine zu filtrierende Fermentationsbrühe auf einer jeweiligen optimalen Reaktionstemperatur zu halten, um dem Erhitzen der Fermentationsbrühe durch z.B. Organismen, die mittels ihres Stoffwechsels Abwärme erzeugen, entgegenzuwirken.

Bei der Ausführungsform von Anspruch 5 können das Filtrationseinrichtungsgehäuse und der Fermenterbehälter voneinander getrennt ausgebildet und angeordnet sein, wobei z.B. der Gehäuseinnenraum der dynamischen Querstromfiltrationseinrichtung und der Behälterinnenraum des Fermenterbehälters über dazwischen angeordnete Leitungen in Fluidverbindung stehen, in welchen z.B. Flusssteuerventile angeordnet sind. Eine solche dezentrale Anordnung kann z.B. aus räumlichen Gründen und/oder aus Gründen einer erforderlichen Anordnung von Antriebsmotoren für die ggf. vorgesehenen Drehwellen Sinn ergeben. Das eigenständige Filtrationseinrichtungsgehäuse ist z.B. ein geschlossenes Gehäuse, mit z.B. einer von einem Gehäusedeckel verschlossenen Gehäuseöffnung für den Zugang zum Gehäuseinnenraum. Bei der Ausführungsform von Anspruch 7 kann z.B. eine sehr kompakte Baueinheit erzielt werden.

Bei der Ausführungsform gemäß Anspruch 8, erste Option, kann der Abwasseranschluss z.B. ein Gully sein, zu welchem und/oder in welchen die (z.B. jeweilige) Permeatabführleitung in Form von z.B. einem Permeatabführschlauch oder einem Permeatabführrohr geführt ist. Im Fall, dass ein Zielprodukt oder Zielprodukte nicht über das Permeat abgeführt wird/werden, sondern in der Fermentationsbrühe verbleibt/verbleiben, kann z.B. ein Sammeln des Permeats nicht erforderlich sein und ein direktes Entsorgen des Permeats über die (z.B. jeweilige) Permeatabführleitung und den Abwasserabschluss erfolgen.

Das Vorsehen eines Feed-Zuführanschlusses gemäß Anspruch 8 und Anspruch 9, jeweils zweite Option, kann z.B. einen im Wesentlichen kontinuierlichen Betrieb der Fermentereinrichtung ermöglichen, wobei kontinuierlich Permeat aus dem Fermenterbehälter abgeführt/abgezogen wird (über die jeweilige Permeatabführleitung) und über den Feed-Zuführanschluss eine der abgeführten Permeat-Menge entsprechende Menge an Fermentationsbrühe in den Fermenterbehälter nachgeführt wird. Im Wesentlichen kontinuierlich schließt hier z.B. auch ein, dass Permeat z.B. in kontinuierlich (z.B. regelmäßig) auftretenden, kurz hintereinanderliegenden Intervallen zugeführt wird gemäß einem in entsprechenden Intervallen erfolgenden Abführen von Permeat.

Im Falle, dass z.B. das Zielprodukt (bzw. die Zielprodukte) über das Permeat abgeführt wird, kann das Permeat in einem Permeatabführbehälter gesammelt werden. Während eine Fermentationsreaktion in der Fermentereinrichtung noch im Gange ist, kann auf diese Weise das Zielprodukt z.B. bereits aufgearbeitet und/oder isoliert werden.

Durch die Bewegung des jeweiligen Filtermembrankörpers kann z.B. per se eine gewisse Durchmischung der Fermentationsbrühe und auch z.B. eine verbesserte Vermischung des über die jeweilige Filtermembran zugeführten Versorgungsgases mit der Fermentationsbrühe erzielt werden. Mit der Ausführungsform gemäß Anspruch 10 kann z.B. das vorgenannte Durchmischen und/oder Vermischen weiter verbessert werden.

Bei dem Fermenter-System gemäß Anspruch 12 kann durch ein gezieltes Einstellen der Porengröße der Filtermembranen z.B. bestimmt werden, ob ein Zielprodukt im Fermenterbehälter und/oder im Filtrationseinrichtungsgehäuse verbleiben oder zusammen mit dem Permeat daraus entfernt werden soll. Darüber hinaus kann mit der Porengröße z.B. die Größe der Gasbläschen des über die jeweilige Filtermembran zugeführten Versorgungsgases beeinflusst und/oder definiert werden.

Die Ausführungsform von Anspruch 15 kann z.B. sinnvoll sein (z.B. in Kombination mit Anspruch 12, erste Alternative), um z.B. instabile oder empfindliche Proteine fortlaufend mit dem Permeat aus der Fermentationsbrühe abzuziehen, sodass diese schnell einer Weiterverarbeitung zuführbar sind. Dies kann auch gelten, wenn bei intervallartigem Abführen der Zielprodukte die Pausen zwischen den Abführintervallen kurz gewählt sind. Ferner kann dies gelten (z.B. in Kombination mit Anspruch 12, zweite Alternative), wenn die Zielprodukte kontinuierlich direkt aus dem Fermenterbehälter abgezogen/abgeführt werden.

Ein kontinuierliches (z.B. permanentes) Abführen der Zielprodukte wird gerne angewandt, wenn z.B. die Zielprodukte, z.B. Metabolismusprodukte, für die in der Fermentationsbrühe vorliegenden Organismen ihrerseits schädlich, z.B. giftig, sind, sodass es bei Nichtabführung zu einem Absterben, z.B. via Eigenvergiftung, der Organismen und damit zu einem Erliegen der Erzeugung der Zielprodukte kommen würde.

Die Erfindung wird nachfolgend anhand von nicht einschränkenden Ausführungsbeispielen mit Bezugnahme auf die Zeichnung weiter erläutert. In der Zeichnung zeigen:
FIG. 1 eine schematische Darstellung einer Fermentereinrichtung gemäß einer Ausführungsform der Erfindung,
FIG. 2 eine vergrößerte, abgeschnittene Querschnittsansicht eines Filtermembrankörpers der Ausführungsform von FIG. 1,
Figuren 3A bis 3F schematische Querschnittsansichten von verschiedenen Ausführungsformen einer Fermentereinrichtung mit einer jeweils in unterschiedlicher Weise daran angeordneten dynamischen Querstromfiltrationseinrichtung und
Figuren 3G und 3H eine Draufsicht von verschiedenen Ausführungsformen einer Fermentereinrichtung, in denen mehrere dynamische Querstromfiltrationseinrichtungen entlang eines Umfangs eines Fermenterbehälters einer Fermentereinrichtung angeordnet sind.

Es versteht sich, dass andere Ausführungsformen benutzt und strukturelle oder logische Änderungen vorgenommen werden können, ohne von dem Schutzumfang der vorliegenden Erfindung abzuweichen. Es versteht sich, dass die Merkmale der hierin beschriebenen Ausführungsformen miteinander kombiniert werden können, sofern nicht spezifisch anders angegeben. Die folgende Beschreibung ist deshalb nicht in einschränkendem Sinne aufzufassen, und der Schutzumfang der vorliegenden Erfindung wird durch die angefügten Ansprüche definiert.

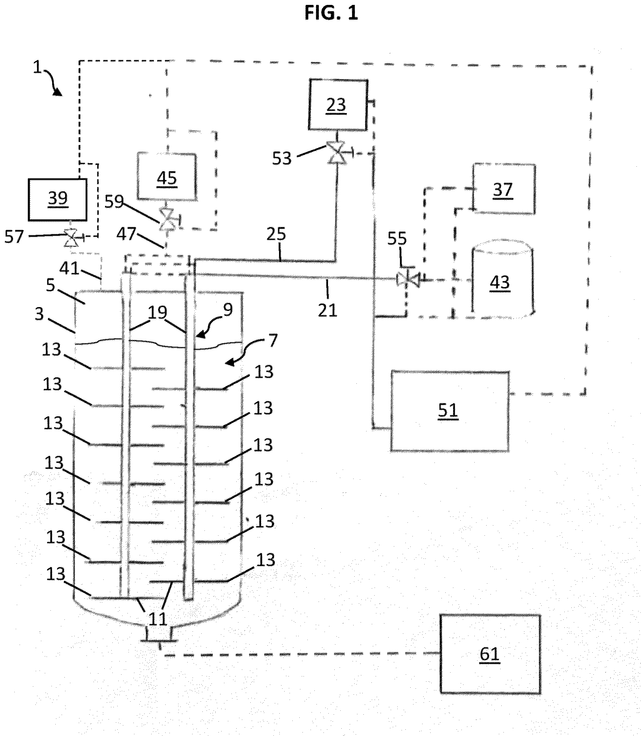

FIG. 1 zeigt eine Fermentereinrichtung 1, aufweisend einen Fermenterbehälter 3 mit einem Behälterinnenraum 5 zur Aufnahme einer Fermentationsbrühe 7.

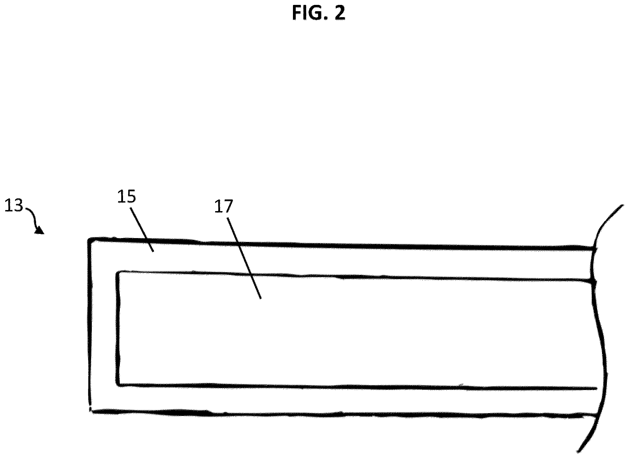

Weiterhin weist die Fermentereinrichtung 1 eine dynamische Querstromfiltrationseinrichtung 9 auf, welche ihrerseits aufweist zwei Filtermembrankörper-Sätze 11 mit jeweils mehreren scheibenförmigen Filtermembrankörpern 13 bzw. Filtermembranscheiben, welche jeweils einen von einer Filtermembran 15 umgebenen Körperinnenraum 17 bzw. Scheibeninnenraum haben (wie z.B. in FIG. 2 veranschaulicht), der mit dem Behälterinnenraum 5 in Fluidkommunikation steht, und welche bewegbar, hier rotationsbewegbar, angeordnet und antreibbar sind mittels einer nicht gezeigten Rotationsantriebsvorrichtung, um im Falle ihrer Bewegung eine Querströmung entlang der zugehörigen Filtermembran 15 in der Fermentationsbrühe 7 zu erzeugen, und welche stapelartig hintereinander sowie in Stapelrichtung im Abstand zueinander angeordnet sind. Weiterhin weist die dynamische Querstromfiltrationseinrichtung 9 dem jeweiligen Filtermembrankörper-Satz 11 zugeordnet, jeweils eine Körperverbindungsleitung 19 bzw. Scheibenverbindungsleitung auf, die mit dem Körperinnenraum 17 des jeweiligen Filtermembrankörpers 13 des zugeordneten Filtermembrankörper-Satzes 11 fluidverbunden ist.

Die Fermentereinrichtung 1 weist weiter eine Permeatabführleitung 21 auf, die mit einer (via durchgezogene Linie 21) oder beiden (via durchgezogene Linie 21 + sich an diese fluchtend anschließende gestrichelte Linie) der zwei Körperverbindungsleitungen 19 verbunden ist zur Abfuhr von Permeat, das durch die Filtermembran 15 des jeweiligen Filtermembrankörpers 13 und über dessen Körperinnenraum 17 in die jeweilige mit der Permeatabführleitung 21 verbundene Körperverbindungsleitung 19 gelangt ist. Die Fermentereinrichtung 1 weist ferner auf eine Versorgungsgasquelle 23, welche über eine Versorgungsleitung 25 mit einer (via durchgezogene Linie 25) oder beiden (via durchgezogene Linie 25 + sich daran horizontal anschließende gestrichelte Linie) der zwei Körperverbindungsleitungen 19 fluidverbunden ist zur Zuführung von Versorgungsgas, welches von in der Fermentationsbrühe 7 enthaltenen Enzymen und/oder Organismen benötigt wird, über die jeweilige mit der Versorgungsgasquelle 23 verbundene Körperverbindungsleitung 19 und den jeweiligen mit dieser fluidverbundenen Körperinnenraum 17 sowie durch die diesen jeweiligen Körperinnenraum 17 umgebende Filtermembran 15 in den Behälterinnenraum 5 (hier direkt in den Behälterinnenraum 5).

Die jeweilige Körperverbindungsleitung 19 ist als hohle Drehwelle ausgebildet, auf welcher die zugeordneten Filtermembrankörper 13 drehfest angeordnet sind, um durch eine Drehung der Drehwelle rotierend bewegt zu werden. Wie in FIG. 1 veranschaulicht, sind die Filtermembrankörper 13 von zwei benachbarten Drehwellen kämmend ineinander eingreifend angeordnet sind.

Die jeweilige Körperverbindungsleitung 19 kann z.B. sowohl mit der wenigstens einen Permeatabführleitung 21 als auch mit der Versorgungsgasquelle 23 fluidverbunden oder fluidverbindbar eingerichtet sein (z.B. über jeweils wenigstens ein Ventil). Alternativ kann die mit der Versorgungsgasquelle 23 fluidverbundene Körperverbindungsleitung 19 nicht mit der Permeatabführleitung 21 verbunden sein und/oder nicht mit der Permeatabführleitung 21 verbindbar eingerichtet sein.

Bei der Ausführungsform von Figur 1 sind zwei Filtermembran-Sätze 11 mit jeweils mehreren Filtermembrankörpern 13 vorgesehen, aber es kann auch nur (z.B. nur wenigstens) ein Filtermembran-Satz 11 mit im Extremfall pro jeweiligem Filtermembran-Satz 11 nur einem Filtermembrankörper 13 vorgesehen sein. Ferner kann auch eine Vielzahl, z.B. 3, 4, 5 oder mehr, von solchen Filtermembran-Sätzen 11 vorgesehen sein.

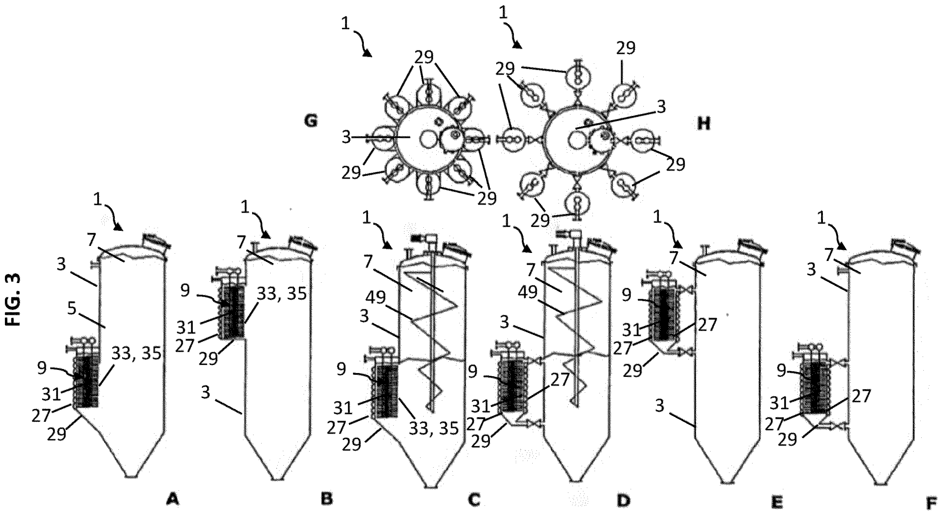

Wie in den Figuren 3A bis 3F dargestellt, kann im Bereich der dynamischen Querstromfiltrationseinrichtung 9 wenigstens eine Kühlvorrichtung 27 angeordnet sein.

Die dynamische Querstromfiltrationseinrichtung 9 kann, wie in den Figuren 3A bis 3F veranschaulicht, ein eigenständiges Filtrationseinrichtungsgehäuse 29 aufweisen, welches einen Gehäuseinnenraum 31 hat, in welchem der jeweilige Filtermembrankörper-Satz 11 angeordnet ist, und welches außen am Fermenterbehälter 3 angebracht ist, wobei der Gehäuseinnenraum 31 mit dem Behälterinnenraum 5 über eine Verbindungsöffnung 33 fluidkommuniziert. Die Verbindungsöffnung 33 kann dabei, wie in den Figuren 3A bis 3C und 3G dargestellt, in einer integralen Verbindungswand 35 ausgebildet sein, welche sowohl Teil einer Behälteraußenwand des Fermenterbehälters 3 als auch eine Gehäuseaußenwand des Filtrationseinrichtungsgehäuses 29 ist bzw. mitdefiniert. Das Filtrationseinrichtungsgehäuse 29 und der Fermenterbehälter 3 können, wie in den Figuren 3D bis 3F und 3H gezeigt, jedoch auch voneinander getrennt ausgebildet und angeordnet sein, wobei der Gehäuseinnenraum 31 der dynamischen Querstromfiltrationseinrichtung 9 und der Behälterinnenraum 5 des Fermenterbehälters 3 über dazwischen angeordnete Leitungen in Fluidverbindung stehen, in welchen Flusssteuerventile angeordnet sind. Bei den Ausführungsformen von Figuren 3A bis 3C stehen aufgrund der integralen Verbindungswand 35 die Filtermembrankörper 13 praktisch direkt mit der Fermentationsbrühe 7 im Kontakt, sodass auch Versorgungsgangs über die Filtermembrankörper 13 direkt in den Behälterinnenraum 5 des Fermenterbehälters 3 zuführbar ist, wohingegen bei den Ausführungsformen von Figuren 3D bis 3F (eher) ein indirekter Kontakt zwischen den Filtermembrankörpern 13 und der im Fermenterbehälter 3 aufgenommenen Filtrationsbrühe 7 vorliegt, sodass auch nur indirekt Versorgungsgas über die Filtermembrankörper 13 in den Behälterinnenraum 5 des Fermenterbehälters 3 zuführbar ist.

Wie in den Figuren 3G und 3H veranschaulicht, kann eine Fermentereinrichtung 1 auch mehrere dynamische Querstromfiltrationseinrichtungen 9 aufweisen, die z.B. entlang ihres Umfangs, z.B. entlang des Querschnittsumfangs ihres Fermenterbehälters 3, angeordnet sind, wodurch ggf. die Filtrationseffizienz weiter verbessert werden kann.

Allerdings kann sich der jeweilige Filtermembrankörper-Satz 11 oder können sich die Filtermembrankörper-Sätze 11, wie in der Ausführungsform von FIG. 1, auch direkt im Behälterinnenraum 5 des Fermenterbehälters 3 befinden.

Die Fermentereinrichtung 1 kann ferner einen Abwasseranschluss 37 aufweisen, mit welchem die wenigstens eine Permeatabführleitung 21 fluidverbunden und/oder fluidverbindbar eingerichtet ist zum Entsorgen, z.B. zum verwertungsfreien Entsorgen, des über die wenigstens eine Permeatabführleitung 21 abgeführten Permeats.

Alternativ (oder ggf. auch zusätzlich zu einem solchen Abwasseranschluss 37) kann die Fermentereinrichtung 1 einen Permeatabführbehälter 43 aufweisen, der mit der wenigstens einen Permeatabführleitung 21 verbunden ist zur Aufnahme des über die Permeatabführleitung 21 abgeführten Permeats.

Darüber hinaus kann die Fermentereinrichtung 1 einen Feed-Zuführanschluss 39 aufweisen, mit welchem der Fermenterbehälter 3 über eine Feed-Zuführleitung 41 verbunden ist zum Zuführen von Brühen-Feed in den Behälterinnenraum 5.

Optional kann die Fermentereinrichtung 1 auch eine Nährstoffsubstratquelle 45 aufweisen, welche mit wenigstens einer oder einer Anzahl oder mit jeder der zwei Körperverbindungsleitungen 19 über eine Nährstoffsubstratzuführleitung 47 fluidverbunden oder fluidverbindbar (über z.B. ein Ventil) eingerichtet ist zur Zuführung von Nährstoffsubstrat, welches von in der Fermentationsbrühe 7 enthaltenen Enzymen und/oder Organismen benötigt wird, über die jeweilige mit der Nährstoffsubstratquelle 45 verbundene Körperverbindungsleitung 19 über den jeweiligen mit dieser fluidverbundenen Körperinnenraum 17 sowie durch die diesen jeweiligen Körperinnenraum 17 umgebende Filtermembran 15 in den Behälterinnenraum 5. Die Nährtstoffsubstratquelle 45 ist z.B. mit der wenigstens einen von der jeweiligen Körperverbindungsleitung 19, die über eine Versorgungsleitung 25 mit der Versorgungsgasquelle 23 fluidverbunden ist, fluidverbunden (z.B. über ein Ventil), um über diese jeweilige Körperverbindungsleitung 19 das Nährstoffsubstrat in den Fermenterbehälter 2 zuführen zu können.

Bei den Ausführungsformen von Figuren 3C und 3D ist die Fermentereinrichtung 1 mit einer Zusatzmischeinrichtung 49 versehen, welche ein Rühr- und/oder Mischwerk hat, das im Fermenterbehälter 3 drehbar und drehend antreibbar ist. Gemäß der Erfindung, und damit bei allen Ausführungsformen, kann z.B. wenigstens einer von dem jeweiligen Filtermembrankörper 13 einen oder mehrere Vorsprünge (hier nicht gezeigt), z.B. eine oder mehre Finnen und/oder eine oder mehrere Flügel, aufweisen, mittels denen eine Durchmischung der Fermentationsbrühe 7 zumindest unterstützt und/oder ohne sonstige Zusatzmischeinrichtung, wie z.B. ohne die oben erläuterte Zusatzmischeinrichtung 49, bewirkt wird.

Die Fermentereinrichtung 1 gemäß der Ausführungsform von Figur 1 weist ferner eine Steuereinrichtung 51 auf. Die Steuereinrichtung 51 ist mit einem Gaszuführsteuerventil 53, welches im Fluidpfad mit der jeweiligen mit der Versorgungsgasquelle 23 verbundenen Körperverbindungsleitung 19 vorliegt, verbunden sein und eingerichtet sein, um das Gaszuführsteuerventil 53 zu steuern zur Steuerung der Versorgungsgaszufuhr in den Fermenterbehälter 3. Die Steuereinrichtung 51 ist weiterhin mit einem Abführventil 55, welches im Fluidpfad mit der jeweiligen mit der Permeatabführleitung 21 verbundenen Körperverbindungsleitung 19 vorliegt, verbunden und eingerichtet, um das Abführventil 55 zu steuern zur Steuerung der Permeatabfuhr an die wenigstens eine Permeatabführleitung 21. Darüber hinaus kann die Steuereinrichtung 51, sofern die Fermentereinrichtung 1 den vorher beschriebenen Feed-Zuführanschluss 39 aufweist, mit einem Feed-Zuführventil 57, welches im Fluidpfad mit der Feed-Zuführleitung 41 vorliegt, verbunden sein und eingerichtet sein, um das Feed-Zuführventil 57 zu steuern zur Steuerung der Brühen-Feed-Zufuhr in den Fermenterbehälter 3. Weiterhin kann die Steuereinrichtung 51, sofern die Fermentereinrichtung 1 die vorher beschriebene Nährstoffsubstratquelle 45 aufweist, mit einem Nährstoffsubstratzuführventil 59, welches im Fluidpfad mit der Nährstoffsubstratzuführleitung 47 vorliegt, verbunden sein und eingerichtet sein, um das Nährstoffsubstratzuführventil 59 zu steuern zur Steuerung der Nährstoffsubstratzufuhr in den Fermenterbehälter 3.

In Figur 1 sowie in den Figuren 3A bis 3F ist ein Fermenter-System mit der Fermentereinrichtung 1 und mit der Fermentationsbrühe 7, die im Fermenterbehälter 3 aufgenommen ist, gezeigt. In der Fermentationsbrühe 7 liegen Zielprodukte, z.B. Metabolismusprodukte, vor, welche eine Produktgröße haben. Die Filtermembranen 15 haben Poren (nicht dargestellt) mit einer Porengröße. Diese Porengröße kann entweder, sofern die Fermentereinrichtung 1 den Permeatabführbehälter 43 aufweist, größer als die Produktgröße sein, sodass die gewonnenen Zielprodukte mit dem Permeat aus der Fermentationsbrühe 7 in den an die Permeatabführleitung 21 angeschlossenen Permeatabführbehälter 43 abführbar sind, oder, sofern die Fermentereinrichtung 1 den Abwasseranschluss 37 aufweist, kleiner als die Produktgröße sein, sodass die gewonnenen Zielprodukte in der Fermentationsbrühe 7 mittels der jeweiligen Filtermembran 15 in der Fermentationsbrühe 7 rückhaltbar sind, wodurch die Fermentationsbrühe 7 mit den Zielprodukten anreicherbar ist. Der Fermenterbehälter 3 kann weiterhin einen Zielproduktentnahmeanschluss 61 aufweisen zur Entnahme von mit Zielprodukten angereicherter Fermentationsbrühe 7 aus dem Fermenterbehälter 3.

Ein Verfahren zum Betreiben der Fermentereinrichtung 1 oder zum Betreiben des Fermenter-Systems weist auf: gesteuertes Zuführen von Versorgungsgas über die jeweilige mit der Versorgungsgasquelle 23 verbundene Körperverbindungsleitung 19 an die in dem Fermenterbehälter 3 aufgenommene Fermentationsbrühe 7 und gesteuertes Abführen von Permeat aus der im Fermenterbehälter 3 aufgenommenen Fermentationsbrühe 7 über die jeweilige mit der Permeatabführleitung 21 verbundene Körperverbindungsleitung 19.

Das gesteuerte Zuführen kann derart erfolgen, dass das Versorgungsgas in Intervallen, z.B. in regelmäßigen Intervallen, der Fermentationsbrühe 7 zugeführt wird.

Das gesteuerte Abführen kann derart erfolgen, dass das Permeat kontinuierlich aus dem Fermenterbehälter 3 abgeführt wird.

Das gesteuerte Zuführen von Versorgungsgas und das gesteuerte Abführen von Permeat können derart erfolgen, dass das Zuführen von Versorgungsgas zeitlich versetzt oderzeitgleich mit dem gesteuerten Abführen von Permeat erfolgt.

Sofern die Fermentereinrichtung 1 den Abwasseranschluss 37 aufweist, kann das gesteuerte Abführen von Permeat derart erfolgen, dass das Permeat über den mit der Permeatabführleitung 21 verbundenen Abwasseranschluss 37 entsorgt wird, z.B. verwertungsfrei entsorgt wird. Alternativ, sofern die Fermentereinrichtung 1 den Permeatabführbehälter 43 aufweist, kann das gesteuerte Abführen von Permeat derart erfolgen, dass das über die Permeatabführleitung 21 abgeführte Permeat in den Permeatabführbehälter 43 zur Weiterverwertung von in dem Permeat enthaltenen Zielprodukten zugeführt wird.

In der Fermentationsbrühe 7 können Zielprodukte, z.B. Metabolismusprodukte, gewonnen werden, welche eine Produktgröße haben, und wobei die Filtermembranen 15 Poren mit einer Porengröße haben. Die Porengröße kann, sofern die Fermentereinrichtung 1 den Permeatabführbehälter 43 aufweist, größer als die Produktgröße sein, sodass die gewonnenen Zielprodukte mit dem Permeat aus der Fermentationsbrühe 7 in einen an die Permeatabführleitung 21 angeschlossenen Permeatabführbehälter 43 abgeführt werden. Alternativ kann die Porengröße, sofern die Fermentereinrichtung 1 den Abwasseranschluss 37 aufweist, kleiner als die Produktgröße sein, sodass die gewonnenen Zielprodukte in der Fermentationsbrühe 7 mittels der jeweiligen Filtermembran 15 in der Fermentationsbrühe 7 zurückgehalten werden, wodurch die Fermentationsbrühe 7 mit den Zielprodukten angereichert wird.

## Patentansprüche

1. Fermentereinrichtung (1), aufweisend:
einen Fermenterbehälter (3) mit einem Behälterinnenraum (5) zur Aufnahme einer Fermentationsbrühe (7),
wenigstens eine dynamische Querstromfiltrationseinrichtung (9), welche aufweist
wenigstens einen Filtermembrankörper-Satz (11) mit wenigstens einem oder mehreren Filtermembrankörpern (13), optional wenigstens einer oder mehreren Filtermembranscheiben, welche/r jeweils einen von einer Filtermembran (15) umgebenen Körperinnenraum (17), optional Scheibeninnenraum, haben/hat, der mit dem Behälterinnenraum (5) in Fluidkommunikation steht, und welche/r bewegbar, optional rotationsbewegbar, angeordnet sind/ist, um im Falle seiner/ihrer Bewegung eine Querströmung entlang der zugehörigen Filtermembran (15) an der Oberfläche der jeweiligen Filtermembran (15) zu erzeugen, und welche optional stapelartig hintereinander sowie in Stapelrichtung im Abstand zueinander angeordnet sind, und,
dem jeweiligen Filtermembrankörper-Satz (11) zugeordnet, jeweils eine Körperverbindungsleitung (19), optional Scheibenverbindungsleitung, die mit dem Körperinnenraum (17) des jeweiligen Filtermembrankörpers (13) des zugeordneten Filtermembrankörper-Satzes (11) fluidverbunden ist,
wenigstens eine Permeatabführleitung (21), die mit wenigstens einer von der jeweiligen Körperverbindungsleitung (19) verbunden ist zur Abfuhr von Permeat, das durch die Filtermembran (15) des jeweiligen Filtermembrankörpers (13) und über dessen Körperinnenraum (17) in die jeweilige mit der wenigstens einen Permeatabführleitung (21) verbundene Körperverbindungsleitung (19) gelangt ist,
**gekennzeichnet durch**
eine Versorgungsgasquelle (23), welche mit wenigstens einer von der jeweiligen Körperverbindungsleitung (19) über eine Versorgungsleitung (25) fluidverbunden ist zur Zuführung von Versorgungsgas, welches von in der Fermentationsbrühe (7) enthaltenen Enzymen und/oder Organismen benötigt wird, über die jeweilige mit der Versorgungsgasquelle (23) verbundene Körperverbindungsleitung (19) und den jeweiligen mit dieser fluidverbundenen Körperinnenraum (17) sowie durch die diesen jeweiligen Körperinnenraum (17) umgebende Filtermembran (15) in den Behälterinnenraum (5).

2. Fermentereinrichtung (1) gemäß Anspruch 1, wobei die jeweilige Körperverbindungsleitung (19) als hohle Drehwelle ausgebildet ist, auf welcher der/die zugeordnete/n Filtermembrankörper (13) drehfest angeordnet ist/sind, um durch eine Drehung der Drehwelle rotierend bewegt zu werden, wobei, optional, die wenigstens eine dynamische Querstromfiltrationseinrichtung (9) mehrere Filtermembrankörper-Sätze (11) mit jeweils mehreren Filtermembrankörpern (13) aufweist, wobei die Filtermembrankörper (13) von zumindest zwei benachbarten Drehwellen kämmend ineinander eingreifend angeordnet sind.

3. Fermentereinrichtung (1) gemäß Anspruch 1 oder 2, wobei die jeweilige Körperverbindungsleitung (19) sowohl mit der wenigstens einen Permeatabführleitung (21) als auch mit der Versorgungsgasquelle (23) fluidverbunden ist, oder wobei zumindest eine, aber nicht alle, von der jeweiligen mit der Versorgungsgasquelle (23) fluidverbundenen Körperverbindungsleitung (19) mit keiner der wenigstens einen Permeatabführleitung (21) verbunden ist und/oder mit keiner der wenigstens einen Permeatabführleitung (21) verbindbar eingerichtet.

4. Fermentereinrichtung (1) gemäß irgendeinem der Ansprüche 1 bis 3, wobei im Bereich der wenigstens einen dynamischen Querstromfiltrationseinrichtung (9) wenigstens eine Kühlvorrichtung (27) angeordnet ist.

5. Fermentereinrichtung (1) gemäß irgendeinem der Ansprüche 1 bis 4, wobei die wenigstens eine dynamische Querstromfiltrationseinrichtung (9) ein eigenständiges Filtrationseinrichtungsgehäuse (29) aufweist, welches einen Gehäuseinnenraum (31) hat, in welchem der jeweilige Filtermembrankörper-Satz (11) angeordnet ist, und welches außen am Fermenterbehälter (3) angebracht ist, wobei der Gehäuseinnenraum (31) mit dem Behälterinnenraum (5) über eine Verbindungsöffnung (33) fluidkommuniziert.

6. Fermentereinrichtung (1) gemäß Anspruch 5, wobei die Verbindungsöffnung (33) in einer integralen Verbindungswand (35) ausgebildet ist, welche sowohl Teil einer Behälteraußenwand des Fermenterbehälters (3) als auch eine Gehäuseaußenwand des Filtrationseinrichtungsgehäuses (29) ist.

7. Fermentereinrichtung (1) gemäß irgendeinem der Ansprüche 1 bis 4, wobei sich der jeweilige Filtermembrankörper-Satz (11) direkt im Behälterinnenraum (5) des Fermenterbehälters (3) befindet.

8. Fermentereinrichtung (1) gemäß irgendeinem der Ansprüche 1 bis 7, ferner aufweisend einen Abwasseranschluss (37), mit welchem die wenigstens eine Permeatabführleitung (21) verbunden ist zum Entsorgen, optional zum verwertungsfreien Entsorgen, des über die wenigstens eine Permeatabführleitung (21) abgeführten Permeats, und/oder ferner aufweisend einen Feed-Zuführanschluss (39), mit welchem der Fermenterbehälter (3) über eine Feed-Zuführleitung (41) verbunden ist zum Zuführen von Brühen-Feed in den Behälterinnenraum (5).

9. Fermentereinrichtung (1) gemäß irgendeinem der Ansprüche 1 bis 7, ferner aufweisend einen Permeatabführbehälter (43), der mit der wenigstens einen Permeatabführleitung (21) verbunden ist zur Aufnahme des über die Permeatabführleitung (21) abgeführten Permeats, und/oder ferner aufweisend einen Feed-Zuführanschluss (39), mit welchem der Fermenterbehälter (3) über eine Feed-Zuführleitung (41) verbunden ist zum Zuführen von Brühen-Feed in den Behälterinnenraum (5).

10. Fermentereinrichtung (1) gemäß irgendeinem der Ansprüche 1 bis 9, wobei wenigstens einer von dem jeweiligen Filtermembrankörper (13) einen oder mehrere Vorsprünge, optional eine oder mehre Finnen und/oder eine oder mehrere Flügel, aufweist, mittels denen eine Durchmischung der Fermentationsbrühe zumindest unterstützt und/oder ohne sonstige Zusatzmischeinrichtung (49) bewirkt wird.

11. Fermentereinrichtung (1) gemäß irgendeinem der Ansprüche 1 bis 10, ferner aufweisend eine Steuereinrichtung (51),
welche mit wenigstens einer Gaszuführpumpe und/oder mit wenigstens einem Gaszuführsteuerventil (53), welche/welches im Fluidpfad mit der jeweiligen mit der Versorgungsgasquelle (23) verbundenen Körperverbindungsleitung (19) vorliegt, verbunden ist und eingerichtet ist, um die jeweilige Gaszuführpumpe und/oder das jeweilige Gaszuführsteuerventil (53) zu steuern zur Steuerung der Versorgungsgaszufuhr in den Fermenterbehälter (3), und/oder
welche mit wenigstens einem Abführventil (55) und/oder mit wenigstens einer Abführpumpe, welches/welche im Fluidpfad mit der jeweiligen mit der wenigstens einen Permeatabführleitung (21) verbundenen Körperverbindungsleitung (19) vorliegt, verbunden ist und eingerichtet ist, um das jeweilige Abführventil (55) und/oder die jeweilige Abführpumpe zu steuern zur Steuerung der Permeatabfuhr an die wenigstens eine Permeatabführleitung (21), und/oder,
sofern in Kombination mit Anspruch 8, zweite Option, oder Anspruch 9, zweite Option, welche mit wenigstens einem Feed-Zuführventil (57) und/oder mit wenigstens einer Feed-Zuführpumpe, welches/welche im Fluidpfad mit der Feed-Zuführleitung (41) vorliegt, verbunden ist und eingerichtet ist, um die jeweilige Feed-Zuführpumpe und/oder das jeweilige Feed-Zuführventil (57) zu steuern zur Steuerung der Brühen-Feed-Zufuhr in den Fermenterbehälter (3).

12. Fermenter-System mit einer Fermentereinrichtung (1) gemäß einem der Ansprüche 1 bis 11 und mit einer Fermentationsbrühe (7), die im Fermenterbehälter (3) aufgenommen ist und in der Zielprodukte, optional Metabolismusprodukte, vorliegen, welche eine Produktgröße haben, und wobei die Filtermembranen (15) Poren mit einer Porengröße haben, welche,
gemäß einer ersten Alternative sofern in Kombination mit Anspruch 9, erste Option, größer als die Produktgröße ist, sodass die gewonnenen Zielprodukte mit dem Permeat aus der Fermentationsbrühe (7) in den an die wenigstens eine Permeatabführleitung (21) angeschlossenen Permeatabführbehälter (43) abführbar sind, oder,
gemäß einer zweiten Alternative sofern in Kombination mit Anspruch 8, erste Option, kleiner als die Produktgröße ist, sodass die gewonnenen Zielprodukte in der Fermentationsbrühe (7) mittels der jeweiligen Filtermembran (15) in der Fermentationsbrühe (7) rückhaltbar sind, wodurch die Fermentationsbrühe (7) mit den Zielprodukten anreicherbar ist, wobei, optional, der Fermenterbehälter (3) einen Zielproduktentnahmeanschluss (61) aufweist zur Entnahme von mit Zielprodukten angereicherter Fermentationsbrühe (7) aus dem Fermenterbehälter (3).

13. Verfahren zum Betreiben einer Fermentereinrichtung (1) gemäß Anspruch 11 oder zum Betreiben eines Fermenter-Systems gemäß Anspruch 12, das eine Fermentereinrichtung (1) gemäß Anspruch 11 aufweist, aufweisend:
gesteuertes Zuführen von Versorgungsgas über die jeweilige mit der Versorgungsgasquelle (23) verbundene Körperverbindungsleitung (19) an eine in dem Fermenterbehälter (3) aufgenommene Fermentationsbrühe (7) und
gesteuertes Abführen von Permeat aus der im Fermenterbehälter (3) aufgenommenen Fermentationsbrühe (7) über die jeweilige mit der wenigstens einen Permeatabführleitung (21) verbundene Körperverbindungsleitung (19).

14. Verfahren gemäß Anspruch 13, wobei das gesteuerte Zuführen derart erfolgt, dass das Versorgungsgas in Intervallen, optional in regelmäßigen Intervallen, der Fermentationsbrühe (7) zugeführt wird.

15. Verfahren gemäß Anspruch 13 oder 14, wobei das gesteuerte Abführen derart erfolgt, dass das Permeat kontinuierlich aus dem Fermenterbehälter (3) abgeführt wird.

16. Verfahren gemäß irgendeinem der Ansprüche 13 bis 15, wobei das gesteuerte Zuführen von Versorgungsgas und das gesteuerte Abführen von Permeat derart erfolgen, dass das Zuführen von Versorgungsgas zeitlich versetzt oder zeitgleich mit dem gesteuerten Abführen von Permeat erfolgt.

17. Verfahren gemäß irgendeinem der Ansprüche 13 bis 16,
wobei, sofern in Kombination mit Anspruch 8, erste Option, das gesteuerte Abführen von Permeat derart erfolgt, dass das Permeat über den mit der wenigstens einen Permeatabführleitung (21) verbundenen Abwasseranschluss (37) entsorgt wird, optional verwertungsfrei entsorgt wird, oder
wobei, sofern in Kombination mit Anspruch 9, erste Option, das gesteuerte Abführen von Permeat derart erfolgt, dass das über die wenigstens eine Permeatabführleitung (21) abgeführte Permeat in den Permeatabführbehälter (43) zur Weiterverwertung von in dem Permeat enthaltenen Zielprodukten zugeführt wird.

18. Verfahren gemäß irgendeinem der Ansprüche 13 bis 17, wobei in der Fermentationsbrühe (7) Zielprodukte, optional Metabolismusprodukte, gewonnen werden, welche eine Produktgröße haben, und wobei die Filtermembranen (15) Poren mit einer Porengröße haben,
welche, sofern in Kombination mit Anspruch 8, erste Option, größer als die Produktgröße ist, sodass die gewonnenen Zielprodukte mit dem Permeat aus der Fermentationsbrühe (7) in einen an die wenigstens eine Permeatabführleitung (21) angeschlossenen Permeatabführbehälter (43) abgeführt werden, oder
welche, sofern in Kombination mit Anspruch 9, erste Option, kleiner als die Produktgröße ist, sodass die gewonnenen Zielprodukte in der Fermentationsbrühe (7) mittels der jeweiligen Filtermembran (15) in der Fermentationsbrühe (7) zurückgehalten werden, wodurch die Fermentationsbrühe (7) mit den Zielprodukten angereichert wird.

## Claims

1. Fermenter device (1), comprising:
a fermenter container (3) with a container internal space (5) for receiving a fermentation broth (7);
at least one dynamic cross-flow filtration device (9) comprising
at least one filter membrane body set (11) with at least one or more filter membrane bodies (13), optionally at least one or more filter membrane discs, which each has/have, surrounded by a filter membrane (15), a body internal space (17), optionally disc internal space, which is in fluid communication with the container internal space (5), and which is/are arranged in a movable, optionally rotationally movable, manner in order to generate a cross-flow along the associated filter membrane (15) at the surface of the respective filter membrane (15) in the event of its/their movement, and which are optionally arranged sequentially in a stack manner as well as at a distance from one another in stacking direction; and,
associated with the respective filter membrane body set (11), a respective body connection line (19), optionally disc connection line, which is fluid-connected to the body internal space (17) of the respective filter membrane body (13) of the associated filter membrane body set (11);
at least one permeate discharge line (21), which is connected to at least one of the respective body connection line (19) for discharging permeate which has passed through the filter membrane (15) of the respective filter membrane body (13) and via its body internal space (17) into the respective body connection line (19) connected to the at least one permeate discharge line (21),
**characterised by**
a supply gas source (23), which is fluid-connected to at least one of the respective body connection lines (19) via a supply line (25) for supplying supply gas, which is required by enzymes and/or organisms contained in the fermentation broth (7), via the respective body connection line (19) connected to the supply gas source (23) and the respective body internal space (17) fluid-connected thereto as well as through the filter membrane (15) surrounding this respective body internal space (17) into the container internal space (5).

2. Fermenter device (1) according to claim 1, wherein the respective body connection line (19) is designed as hollow rotary shaft on which the associated filter membrane body/bodies (13) is/are arranged in a rotationally fixed manner in order to be rotationally moved by a rotation of the rotary shaft, wherein, optionally, the at least one dynamic cross-flow filtration device (9) has a plurality of filter membrane body sets (11) each comprising a plurality of filter membrane bodies (13), wherein the filter membrane bodies (13) of at least two adjacent rotary shafts are arranged to mesh with one another.

3. Fermenter device (1) according to claim 1 or 2, wherein the respective body connection line (19) is fluid-connected both to the at least one permeate discharge line (21) and to the supply gas source (23), or wherein at least one, but not all, of the respective body connection line (19) fluid-connected to the supply gas source (23) is connected to none of the at least one permeate discharge line (21) and/or is arranged connectable to none of the at least one permeate discharge line (21).

4. Fermenter device (1) according to any one of claims 1 to 3, wherein at least one cooling device (27) is arranged in the region of the at least one dynamic cross-flow filtration device (9).

5. Fermenter device (1) according to any one of claims 1 to 4, wherein the at least one dynamic cross-flow filtration device (9) comprises an independent filtration device housing (29) which has a housing internal space (31) in which the respective filter membrane body set (11) is arranged and which is attached on the outside of the fermenter container (3), wherein the housing internal space (31) fluid-communicates with the container internal space (5) via a connection opening (33).

6. Fermenter device (1) according to claim 5, wherein the connection opening (33) is formed in an integral connecting wall (35), which is both part of a container outer wall of the fermenter container (3) and a housing outer wall of the filtration device housing (29).

7. Fermenter device (1) according to any one of claims 1 to 4, wherein the respective filter membrane body set (11) is located directly in the container internal space (5) of the fermenter container (3).

8. Fermenter device (1) according to any one of claims 1 to 7, further comprising a waste water connection (37), to which the at least one permeate discharge line (21) is connected for disposing, optionally for disposing without utilisation, of the permeate discharged via the at least one permeate discharge line (21), and/or further comprising a feed supply connection (39), to which the fermenter container (3) is connected via a feed supply line (41) for supplying broth feed into the container internal space (5).

9. Fermenter device (1) according to any one of claims 1 to 7, further comprising a permeate discharge container (43) which is connected to the at least one permeate discharge line (21) for receiving the permeate discharged via the permeate discharge line (21), and/or further comprising a feed supply connection (39) to which the fermenter container (3) is connected via a feed supply line (41) for supplying broth feed into the container internal space (5).

10. Fermenter device (1) according to any one of claims 1 to 9, wherein at least one of the respective filter membrane body (13) comprises one or more projections, optionally one or more fins and/or one or more wings, by means of which mixing of the fermentation broth is at least supported and/or effected without any other additional mixing device (49).

11. Fermenter device (1) according to any one of claims 1 to 10, further comprising a control device (51)
which is connected to at least one gas supply pump and/or to at least one gas supply control valve (53), which is/are present in the fluid path with the respective body connection line (19) connected to the supply gas source (23), and is arranged to control the respective gas supply pump and/or the respective gas supply control valve (53) for controlling the supply gas supply into the fermenter container (3), and/or
which is connected to at least one discharge valve (55) and/or to at least one discharge pump, which is/are present in the fluid path with the respective body connection line (19) connected to the at least one permeate discharge line (21), and is arranged to control the respective discharge valve (55) and/or the respective discharge pump for controlling the discharge of permeate to the at least one permeate discharge line (21), and/or,
if in combination with claim 8, second option, or claim 9, second option, which is connected to at least one feed supply valve (57) and/or to at least one feed supply pump which is/are present in the fluid path with the feed supply line (41) and is arranged to control the respective feed supply pump and/or the respective feed supply valve (57) for controlling the broth feed supply into the fermenter container (3).

12. Fermenter system comprising a fermenter device (1) according to any one of claims 1 to 11 and comprising a fermentation broth (7) which is received in the fermenter container (3) and in which target products, optionally metabolism products, are present which have a product size, and wherein the filter membranes (15) have pores with a pore size which,
according to a first alternative if in combination with claim 9, first option, is larger than the product size, so that the obtained target products are dischargeable with the permeate from the fermentation broth (7) into the permeate discharge container (43) connected to the at least one permeate discharge line (21), or,
according to a second alternative if in combination with claim 8, first option, is smaller than the product size, so that the obtained target products in the fermentation broth (7) are retainable in the fermentation broth (7) by means of the respective filter membrane (15), whereby the fermentation broth (7) is enrichable with the target products, wherein, optionally, the fermenter container (3) comprises a target product removal connection (61) for removing fermentation broth (7) enriched with target products from the fermenter container (3).

13. Method for operating a fermenter device (1) according to claim 11 or for operating a fermenter system according to claim 12, comprising a fermenter device (1) according to claim 11, comprising:
controlled supply of supply gas via the respective body connection line (19) connected to the supply gas source (23) to a fermentation broth (7) received in the fermenter container (3); and
controlled discharge of permeate from the fermentation broth (7) received in the fermenter container (3) via the respective body connection line (19) connected to the at least one permeate discharge line (21).

14. Method according to claim 13, wherein the controlled supply is carried out such that the supply gas is supplied to the fermentation broth (7) at intervals, optionally at regular intervals.

15. Method according to claim 13 or 14, wherein the controlled discharge is carried out such that the permeate is continuously discharged from the fermenter container (3).

16. Method according to any one of claims 13 to 15, wherein the controlled supply of supply gas and the controlled discharge of permeate are carried out such that the supply of supply gas is carried out with a time shift or simultaneously with the controlled discharge of permeate.

17. Method according to any one of claims 13 to 16,
wherein, if in combination with claim 8, first option, the controlled discharge of permeate is carried out such that the permeate is disposed of via the waste water connection (37) connected to the at least one permeate discharge line (21), optionally disposed of without utilisation, or
wherein, if in combination with claim 9, first option, the controlled discharge of permeate is carried out such that the permeate discharged via the at least one permeate discharge line (21) is supplied into the permeate discharge container (43) for further utilisation of target products contained in the permeate.

18. Method according to any one of claims 13 to 17, wherein in the fermentation broth (7) target products, optionally metabolism products, are obtained which have a product size, and wherein the filter membranes (15) have pores having a pore size
which, if in combination with claim 8, first option, is larger than the product size, so that the obtained target products are discharged with the permeate from the fermentation broth (7) into a permeate discharge container (43) connected to the at least one permeate discharge line (21), or
which, if in combination with claim 9, first option, is smaller than the product size, so that the obtained target products in the fermentation broth (7) are retained in the fermentation broth (7) by means of the respective filter membrane (15), whereby the fermentation broth (7) is enriched with the target products.

## Revendications

1. Dispositif de fermentation (1), comprenant:
un réservoir de fermentation (3) avec un espace intérieur de réservoir (5) pour recevoir un bouillon de fermentation (7),
au moins un dispositif de filtration dynamique à courant croisé (9), comprenant
au moins un ensemble de corps de membrane filtrante (11) comprenant au moins un ou plusieurs corps de membrane filtrante (13), optionnellement au moins un ou plusieurs disques de membrane filtrante, qui a/ont un espace intérieur de corps (17) respectif, optionnellement un espace intérieur de disque, entouré par une membrane filtrante (15), qui est en communication fluidique avec l'espace intérieur de réservoir (5), et qui est/sont disposé(s) de manière mobile, optionnellement mobile en rotation, afin de générer, dans le cas de son/leur mouvement, un courant croisé le long de la membrane filtrante (15) respective sur la surface de la membrane filtrante (15) respective, et qui sont disposés optionnellement en forme de pile les uns derrière les autres ainsi qu'à distance les uns des autres dans le sens de la pile, et,
associé à l'ensemble de corps de membrane filtrante (11) respectif, respectivement une conduite de connexion de corps (19), optionnellement une conduite de connexion de disques, qui est reliée par fluide à l'espace intérieur de corps (17) du corps de membrane filtrante (13) respectif de l'ensemble de corps de membrane filtrante (11) associé,
au moins une conduite de déchargement de perméat (21) reliée à au moins une conduite de connexion de corps respective (19) pour décharger de perméat qui est passé à travers la membrane filtrante (15) du corps de membrane filtrante (13) respectif et par son intérieur de corps (17) dans la conduite de connexion de corps (19) respective reliée à ladite au moins une conduite de déchargement de perméat (21),
**caractérisé par**
une source de gaz d'alimentation (23) qui est reliée par fluide à au moins l'une de conduite de connexion de corps (19) respective par une conduite d'alimentation (25) pour l'alimentation en gaz d'alimentation, qui est nécessaire aux enzymes et/ou aux organismes contenus dans le bouillon de fermentation (7), par la conduite de connexion de corps (19) respective reliée à la source de gaz d'alimentation (23) et l'espace intérieur de corps (17) respectif relié par fluide à celle-ci, ainsi qu'à travers la membrane filtrante (15) entourant cet espace intérieur de corps (17) respectif, dans l'espace intérieur de réservoir (5).

2. Dispositif de fermentation (1) selon la revendication 1, dans lequel la conduite de connexion de corps (19) respective est réalisée sous la forme d'un arbre rotatif creux sur lequel le/les corps de membrane filtrante (13) associé(s) est/sont disposé(s) de manière immobile en rotation afin d'être déplacé(s) en rotation par une rotation de l'arbre rotatif, dans lequel, optionnellement, le au moins un dispositif de filtration dynamique à courant croisé (9) comprend plusieurs ensembles de corps de membrane filtrante (11) avec respectivement plusieurs corps de membrane filtrante (13), les corps de membrane filtrante (13) d'au moins deux arbres rotatifs adjacents étant disposés de manière à s'engrener les uns dans les autres.

3. Dispositif de fermentation (1) selon la revendication 1 ou 2, dans lequel la conduite de connexion de corps (19) respective est reliée par fluide à la fois à la au moins une conduite de déchargement de perméat (21) et à la source de gaz d'alimentation (23), ou dans lequel au moins une, mais pas toutes, de conduite de connexion de corps (19) respective reliée par fluide à la source de gaz d'alimentation (23) n'est reliée à aucune de la au moins une conduite de déchargement de perméat (21) et/ou n'est réalisée reliable à aucune de la au moins une conduite de déchargement de perméat (21).

4. Dispositif de fermentation (1) selon l'une quelconque des revendications 1 à 3, dans lequel au moins un dispositif de refroidissement (27) est disposé dans la région d'au moins un dispositif de filtration dynamique à courant croisé (9).

5. Dispositif de fermentation (1) selon l'une quelconque des revendications 1 à 4, dans lequel le au moins un dispositif de filtration dynamique à courant croisé (9) comprend un boitier de dispositif de filtration (29) autonome qui a un espace intérieur de boitier (31) dans lequel l'ensemble de corps de membrane filtrante (11) respectif est disposé et qui est monté à l'extérieur du réservoir de fermentation (3), l'espace intérieur de boitier (31) communiquant par fluide avec l'espace intérieur de réservoir (5) par une ouverture de connexion (33).

6. Dispositif de fermentation (1) selon la revendication 5, dans lequel l'ouverture de connexion (33) est formée dans une paroi de connexion (35) intégrale qui fait partie à la fois d'une paroi extérieure de réservoir du réservoir de fermentation (3) et d'une paroi extérieure de boitier du boitier de dispositif de filtration (29).

7. Dispositif de fermentation (1) selon l'une quelconque des revendications 1 à 4, dans lequel l'ensemble de corps de membrane filtrante (11) respectif est situé directement dans l'espace intérieur de réservoir (5) du réservoir de fermentation (3).

8. Dispositif de fermentation (1) selon l'une quelconque des revendications 1 à 7, comprenant en outre un raccordement d'eaux usées (37) auquel est relié la au moins une conduite de déchargement de perméat (21) pour décharger, optionnellement décharger sans usage, le perméat déchargé par la au moins une conduite de déchargement de perméat (21), et/ou comprenant en outre un raccordement d'alimentation de feed (39) auquel est relié le réservoir de fermentation (3) par une conduite d'alimentation de feed (41) pour l'alimentation en bouillon de feed à l'intérieur de réservoir (5).

9. Dispositif de fermentation (1) selon l'une quelconque des revendications 1 à 7, comprenant en outre un réservoir de déchargement de perméat (43) relié à la au moins une conduite de déchargement de perméat (21) pour recevoir le perméat déchargé via la conduite de déchargement de perméat (21), et/ou comprenant en outre un raccordement d'alimentation de feed (39) auquel le réservoir de fermentation (3) est relié via une conduite d'alimentation de feed (41) pour l'alimentation en bouillon de feed à l'intérieur de réservoir (5).

10. Dispositif de fermentation (1) selon l'une quelconque des revendications 1 à 9, dans lequel au moins l'un des corps de membrane filtrante (13) respectif comprend une ou plusieures protrusions, optionnellement un ou plusieurs ailerons et/ou une ou plusieurs ailes, au moyen desquelles un mélange du bouillon de fermentation est au moins assisté et/ou est effectué sans autre dispositif de mélange supplémentaire (49).

11. Dispositif de fermentation (1) selon l'une quelconque des revendications 1 à 10, comprenant en outre un dispositif de commande (51),
qui est relié à au moins une pompe d'alimentation en gaz et/ou à au moins une vanne de commande d'alimentation en gaz (53) qui est présente dans le trajet de fluide avec la conduite de connexion de corps (19) respective reliée à la source de gaz d'alimentation (23), et qui est agencé pour commander la pompe d'alimentation en gaz respective et/ou la vanne de commande d'alimentation en gaz (53) respective pour commander l'alimentation en gaz d'alimentation dans le réservoir de fermentation (3), et/ou
qui est relié à au moins une vanne de déchargement (55) et/ou à au moins une pompe de déchargement qui est présente dans le trajet de fluide avec la conduite de connexion de corps (19) respective reliée à la au moins une conduite de déchargement de perméat (21), et est agencé pour commander la vanne de déchargement (55) respective et/ou la pompe de déchargement respective pour commander le déchargement de perméat à la au moins une conduite de déchargement de perméat (21), et/ou,
si en combinaison avec la revendication 8, deuxième option, ou la revendication 9, deuxième option, qui est relié à au moins une vanne d'alimentation de feed (57) et/ou à au moins une pompe d'alimentation de feed qui est présente dans le trajet de fluide avec la conduite d'alimentation de feed (41) et qui est agencé pour commander la pompe d'alimentation de feed respective et/ou la vanne d'alimentation de feed (57) respective pour commander l'alimentation de bouillon de feed dans le réservoir de fermentation (3).

12. Système de fermentation comprenant un dispositif de fermentation (1) selon l'une quelconque des revendications 1 à 11 et comprenant un bouillon de fermentation (7) qui est reçu dans le réservoir de fermentation (3) et dans lequel sont présents des produits cibles, optionnellement des produits de métabolisation, qui ont une taille de produit, et dans lequel les membranes filtrantes (15) ont des pores ayant une taille de pore qui,
selon une première alternative, si en combinaison avec la revendication 9, première option, est supérieure à la taille du produit, de sorte que les produits cibles obtenus sont déchargeables avec le perméat du bouillon de fermentation (7) au réservoir de déchargement de perméat (43) raccordé à la au moins une conduite de déchargement de perméat (21), ou,
selon une deuxième alternative, si en combinaison avec la revendication 8, première option, est plus petite que la taille du produit, de sorte que les produits cibles obtenus dans le bouillon de fermentation (7) peuvent être retenus dans le bouillon de fermentation (7) au moyen de la membrane filtrante (15) respective, ce qui permet d'enrichir le bouillon de fermentation (7) avec les produits cibles, dans lequel, optionnellement, le réservoir de fermentation (3) comprend un raccordement de prélèvement de produits cibles (61) pour prélever le bouillon de fermentation (7) enrichi en produits cibles du réservoir de fermentation (3).

13. Procédé pour faire fonctionner un dispositif de fermentation (1) selon la revendication 11 ou pour faire fonctionner un système de fermentation selon la revendication 12, qui comprend un dispositif de fermentation (1) selon la revendication 11, comprenant:
alimentation contrôlée en gaz d'alimentation, par la conduite de connexion de corps (19) respective reliée à la source de gaz d'alimentation (23), à un bouillon de fermentation (7) reçu dans le réservoir de fermentation (3), et
déchargement commandé de perméat du bouillon de fermentation (7) reçu dans le réservoir de fermentation (3) par la conduite de connexion de corps (19) respective reliée à la au moins une conduite de déchargement de perméat (21).

14. Procédé selon la revendication 13, dans lequel l'alimentation contrôlée est telle que le gaz d'alimentation est fourni au bouillon de fermentation (7) à des intervalles, optionnellement à des intervalles réguliers.

15. Procédé selon la revendication 13 ou 14, dans lequel le déchargement contrôlé est effectué de telle sorte que le perméat est déchargé en continu du réservoir de fermentation (3).

16. Procédé selon l'une quelconque des revendications 13 à 15, dans lequel l'alimentation commandée en gaz d'alimentation et le déchargement commandée du perméat sont effectuées de telle sorte que l'alimentation en gaz d'alimentation est décalée dans le temps ou simultanée au déchargement commandé du perméat.

17. Procédé selon l'une quelconque des revendications 13 à 16,
dans lequel, si en combinaison avec la revendication 8, première option, le déchargement contrôlé du perméat est effectué de telle sorte que le perméat est déchargé via le raccordement d'eaux usées (37) relié à la au moins une conduite de déchargement de perméat (21), optionnellement déchargé sans utilisation, ou
dans lequel, si en combinaison avec la revendication 9, première option, le déchargement contrôlé du perméat est effectué de telle sorte que le perméat déchargé par la au moins une conduite de déchargement de perméat (21) est alimenté dans le réservoir de déchargement de perméat (43) pour utilisation ultérieure de produits cibles contenus dans le perméat.

18. Procédé selon l'une quelconque des revendications 13 à 17, dans lequel des produits cibles, optionnellement des produits de métabolisation, sont obtenus dans le bouillon de fermentation (7), qui ont une taille de produit, et dans lequel les membranes filtrantes (15) ont des pores ayant une taille de pore,
qui, si en combinaison avec la revendication 8, première option, est supérieure à la taille du produit, de sorte que les produits cibles obtenus sont déchargés avec le perméat du bouillon de fermentation (7) dans un réservoir de déchargement de perméat (43) raccordé à la au moins une conduite de déchargement de perméat (21), ou
qui, si en combinaison avec la revendication 9, première option, est inférieure à la taille du produit, de sorte que les produits cibles obtenus dans le bouillon de fermentation (7) sont retenus dans le bouillon de fermentation (7) au moyen de la membrane filtrante (15) respective, enrichissant ainsi le bouillon de fermentation (7) avec les produits cibles.
